# EUROPEAN PATENT SPECIFICATION

(11) **EP 0 917 877 B1**
(45) Date of publication and mention of the grant of the patent: **17.09.2008**
(21) Application number: 97930787.3
(22) Date of filing: 14.07.1997
(51) Int. Cl.: A61K 35/14, A61P 31/12

(54) **REMEDIES/PREVENTIVES FOR VIRAL INFECTIONS, PROCESS FOR PREPARING THE SAME, AND METHOD FOR PREVENTING/TREATING VIRAL INFECTIONS**
MITTEL ZUR HEILUNG BZW. VORBEUGUNG VIRALER INFEKTIONEN, VERFAHREN ZU DEREN HERSTELLUNG SOWIE VERFAHREN ZUR VORBEUGUNG BZW. HEILUNG VIRALER INFEKTIONEN
REMEDES/AGENTS PROPHYLACTIQUES POUR INFECTIONS VIRALES, LEURS PROCEDES DE PRODUCTION, ET PROCEDE DE PREVENTION/TRAITEMENT D'INFECTIONS VIRALES

(30) Priority: 15.07.1996 JP 20429496
(43) Date of publication of application: 26.05.1999
(73) Proprietor: Lymphotec Inc., Tokyo (JP)
(72) Inventor: SEKINE, Teruaki, Koto-ku, Tokyo 135 (JP)
(74) Representative: Neilson, Martin Mark
(86) International application number: PCT/JP1997/002438
(87) International publication number: WO 1998/002175

(56) References cited:
- JP-A- 3 080 076
- US-A- 5 443 983
- KAST W M ET AL: "Treatment with monoclonal anti-CD3 antibody protects against lethal Sendai virus infection by induction of natural killer cells." JOURNAL OF IMMUNOLOGY (BALTIMORE, MD. : 1950) 1 OCT 1990, vol. 145, no. 7, 1 October 1990 (1990-10-01), pages 2254-2259, XP002294779 ISSN: 0022-1767
- NATURE MEDICINE, Vol. 1, No. 4, (1995), SCOTT KOENIG et al., "Transfer of HIV-1-Specific Cytotoxic T Lymphocytes to an AIDS Patient Leads to Selection for Mutant HIV Variants and Subsequent Disease Progression", pages 330-336.
- THE NEW ENGLAND JOURNAL OF MEDICINE, Vol. 333, No. 16, (1995), ELIZABETH A. WALTER et al., "Reconstitution of Cellular Immunity Against Cytomegalovirus in Recipients of Allogeneic Bone Marrow by Transfer of T-Cell Clones from the Donor", pages 1038-1044.
- HO ET AL: "A phase 1 study of adoptive transfer of autologous CD8 + T lymphocytes in patients with acquired immunodeficiency syndrome (AIDS)-related complex or AIDS" BLOOD, vol. 81, no. 8, 15 April 1993 (1993-04-15), pages 2093-2101,

## Description

### TECHNICAL FIELD

This invention relates to remedies or preventives for viral infections containing, as the main ingredient, autologous lymphocytes cultivated in a culture medium containing an anti-CD3 antibody and interleukin-2 made into a solid phase, which can be used for not only preventing or treating the viral infections so as to improve the therapeutic or preventive effects on the viral infections, but also sufficiently preventing immunodeficiency or immunosuppression and postoperative or post-transplantation viral infections.

### BACKGROUND ART

Living bodies are protected from various infections and cancers by their own immunity system. In general, humoral immunity and cellular immunity have been known as the immunity system. Complements being one kind of serum proteins, lysozyme and antibody bestow the humoral immunity, and macrophage and cells such as NK cells and T cells bestow the cellular immunity. The cellular immunity brought about mainly by the NK cells and T cells seems to be important to the immune reaction to cancers.

Cells are sorted according to their surface markers appearing on the surfaces thereof. On the mature T cell, CD3 emerges as the surface marker. Further, CD8 appears on a killer T cell having antitumor activity and antiviral activity, and CD4 appears on a helper T cell which are closely related to the activation of cells having various immune systems. Besides, the mature T cells bear T cell receptors which have high affinity relative to specific antigens. The T cells bear only a single type of T cell receptors through which antigen-specific T cells are activated. Furthermore, different sorts of T cell receptors which are very diverse exit within a living body.

Incidentally, the complements which singularly inactivate viruses, lysozymes, and macrophages which eat greedily the complements are important to protection against viral infections at the first stage. From a few days after getting a viral infection, viral-specific antibodies are rapidly increased, and consequently, fulfill the important function to protect against the viral infections. As the viral-specific antibody, there have been known a neutralizing antibody serving to inactivate the viruses and a specific antibody capable of exerting ADCC activity having a function of specifically removing cells infected with viruses with the cooperation of T cells and so forth, though the ADCC activity per se cannot make viruses inactive. Therefore, the viral-infection protective reaction takes place principally by the humoral immunity and cellular immunity. Thus, the defensive reaction of the living body against the cancer is remarkably different from that against the viral infections, although both the reactions occur through the immunity system.

The virus which is essentially extraneous relative to the living body has the nature of living with its host cell to proliferate. From one infective virus, thousands to tens of thousands of virus particulars are brought forth at once with explosive speed. However, the cancer naturally brought about due to mutation of host-derived cells requires twenty hours or thereabout at the earliest to cause binary fission. Of the various viruses, there are viruses lurking in infected cells of the living body, with the result of which the living body is latently infected therewith. As the latently infective viruses, there have been known various herpesviruses and retroviruses. These viruses frequently evolve to pathogenic viruses when the immunity power of the host is weakened.

As noted above, the origins of the cancer and affected with a viral infection are wholly different in living body defensive mechanism and proliferating speed and mechanism. Accordingly, the therapy in the treatment for cancer is not necessarily effective in the preventive or therapeutic treatment for viral infections. In actual fact, an anticancer drug clinically used differs from an antiviral agent in most cases. Antitumor activation by stimulation of lymphocytes, particularly, T cells and NK cells, of a cancer patient by interleukin-2 was reported by Rosenberg (New Eng. J. Med., 319, 1678(1988)). The inventors of the present invention have already disclosed a stimulation method for proliferation of lymphocytes, by which the lymphocytes of the cancer patient are stimulated with a coagulated anti-CD3 antibody and interleukin-2, consequently to be proliferated (Japanese Patent Application Public Disclosure No. 03-80076(A)).

A remedy for cancer by use of the aforesaid lymphocytes has been reported (Human Cell, 7, pp.121-123(1994). Further, Kliona M. Rooney et al. have reported that lymphocytes derived from a donor of the bone marrow, which are proliferated by stimulation with the interleukin-2 and Epstein-Barr virus transformation lymphocytes, are effective in proliferating the lymphocytes participating with Epstein-Barr viruses brought forth in bone marrow transplantation (Lancet, 345, 9(1995)). Also, Elizabeth A. Walter et al. have reported that lymphocytes derived from a donor of the bone marrow, which are proliferated by stimulation with the anti-CD3 antibody, fibroblast infected with cytomegalovirus and interleukin-2 are effective in remedying cytomegalovirus infections in bone marrow transplantation (New Eng. J. Med., 333(16), 1038(1995)). However, a remedy for viral infections by use of autologous lymphocytes of a patient, which are proliferated by being stimulated with the coagulated anti-CD3 antibody and interleukin-2 has not at all been reported so far. Scott et al. carried on the research concerning a remedy of HIV by using lymphocytes showing peculiarity relative to HIV, which are prepared from the lymphocytes derived from the patient by use of PHA, interleukin-2, feeder cells and OKT-3, but has not succeeded in remedying HIV infections by that remedy (Nature Medicine, 1(4), pp. 330-336 (1995)).

However, Japanese Patent Appln. Disclosure No. 03-80076(A) is concerned with the remedy for cancer by use of the lymphocytes which are proliferated by being stimulated with the CD3 antibody and interleukin-2, but does not reveal whether or not the lymphocytes proliferated by being stimulated with the CD3 antibody and interleukin-2 are efficacious against infectious cancers.

Likewise, Human Cell merely discloses the remedy for cancer by use of the lymphocytes proliferated by being stimulated with the CD3 antibody and interleukin-2, but does not elucidate that the lymphocytes proliferated by being stimulated with the CD3 antibody and interleukin-2 are efficacious against infectious cancers.

Although Kliona M. Rooney et al. reported that the lymphocytes derived from the donor of the bone marrow proliferated by stimulation with the virus transformation lymphocytes are effective in proliferating the lymphocytes participating with Epstein-Barr viruses brought forth in bone marrow transplantation, the lymphocytes used therefor were not derived from the patient, but obtained from the donor of the bone marrow and that of health, who does not contract any infection. That is, it is not evident from the report that the lymphocytes, which are obtained by proliferating with stimulation the lymphocytes derived from the patient contracting the infections and showing the symptoms of the infections, are efficacious against the infections. Moreover, use of an anti-CD3 antibody which coagulates in the process of stimulation to proliferate the lymphocytes is not confirmed in the report, and in conclusion, the method for stimulating and proliferating the lymphocytes as proposed in the report is fundamentally distinct from that using autologous lymphocytes.

Also, the lymphocytes used in the report of Elizabeth A. Walter, disclosing that lymphocytes derived from a donor of the bone marrow proliferated by being stimulated with the anti-CD3 antibody, fibroblast infected with cytomegalovirus and interleukin-2 are effective in remedying cytomegalovirus infections in bone marrow transplantation, were not derived from the patient, but obtained from the donor of the bone marrow and that of health, who does not contract any infection. That is, it is not evident from the report that the lymphocytes, which are obtained by proliferating with stimulation the lymphocytes derived from the patient contracting the infection and showing the symptoms of the infections, are effective against the infections. Moreover, use of an anti-CD3 antibody which coagulates in the process of stimulation to proliferate the lymphocytes is not confirmed in the report, and consequently, the method for stimulating and proliferating the lymphocytes as proposed in this report is fundamentally distinct from that using autologous lymphocytes. Therefore, it can be said that the remedy proposed in this report is unsuitable for a patient contracting a viral infection.

EP 409655 only discloses an *in vitro* method of proliferation of certain lymphocytic cellular sub-populations by contacting peripheral blood lymphocytes with an effective amount of an anti-CD3 antibody and IL-2.

An object of the present invention is to provide remedies or preventives for the treatment or prevention of viral infections, which can more improve the effects of treatment and prevention of viral infections, effectively prevent postoperative or post-transplantation viral infections, and remarkably increase the efficiency of proliferating lymphocytes.

Another object of the invention is to provide, in the concrete, remedies or preventives for the treatment or prevention of viral infections, which contain, as the main ingredient, autologous lymphocytes proliferated by being cultivated in a culture medium containing an anti-CD3 antibody and interleukin-2 made into a solid phase.

Still another object of the invention is to provide remedies or preventives for viral infections, which are markedly efficacious for viral infections in patients with immunodeficiency or immunosuppression.

Lymphocytes collected from a patient contracting at viral infection are cultivated in a culture medium made into a solid phase, which contains an anti-CD3 antibody and interleukin-2 so as to be proliferated immediately after the cultivation. After the lapse of about two weeks, the number of lymphocytes becomes about 1000 to 10000 times.

### DISCLOSURE OF THE INVENTION

The remedies or preventives for viral infections according to the present invention are featured in that the aforementioned autologous lymphocytes are proliferated by being cultivated in the culture medium containing the anti-CD3 antibody and interleukin-2 made into a solid phase.

### THE BEST MODE FOR CARRYING OUT THE INVENTION

The remedies or preventives for viral infections according to the present invention contains, as the main ingredient, autologous lymphocytes cultivated in a culture medium containing an anti-CD3 antibody and interleukin-2 made into a solid phase. The viral infections specified herein are defined as a state in which a patient has already contracted a viral infection in treatment or prevention therefor or an immunosuppression state in which a patient is exposed to the possibility of contracting the viral infection.

Generally, cells are sorted according to their surface markers appearing on the surfaces thereof. On the mature T cell, CD3 emerges as the surface marker. Further, CD8 appears on a killer T cell having antitumor activity and antiviral activity, and CD4 appears on a helper T cell, which are closely related to the activation of cells having various immune systems. Besides, the mature T cells bear T cell receptors which have high affinity relative to specific antigens. The T cells bear only a single type of T cell receptors through which antigen-specific T cells are activated. Furthermore, different sorts of T cell receptors which are very diverse exit within a living body.

On the other hand, as a viral infection, there are enumerated infections caused by viruses belonging to various herpesvirus groups, which are typified by cytomegalovirus, Esstein-Barr virus, herpes simplex virus, and varicellazoster virus, viruses belonging to various retrovirus groups, which are typified by human immunodeficiency virus and HTLV, and various hepatitis viruses typified by hepatitis A virus, hepatitis B virus, hepatitis C virus, hepatitis D virus, hepatitis E virus, hepatitis F virus, and hepatitis G virus, and infections due to causal bacteria such as influenza viruses. The remedies or preventives for viral infections according to this invention are especially efficacious against the viral infections caused by the viruses of the herpesvirus groups, but the application of the present invention is by no means limited to the viruses enumerated.

In particular, the present invention is effective in remedying patients with immunodeficiency or immunosuppression. The immunodeficiency belongs to either a congenital immunodeficiency syndrome or an acquired immunodeficiency syndrome. The congenital immunodeficiency is typified by the Wiskott-Aldrich syndrome, and the acquired immunodeficiency is typified by AIDS and ARC. The immunosuppression is caused in the state of giving immunosuppressing remedial agents for organ transplantation or bone marrow transplantation, bearing tumor, getting scaled, following a surgical operation, or producing side effects on immune cells due to medication of remedial agents. The causes for immunodeficiency or immunosuppression are not limited thereto. The remedies or preventives according to this invention are especially effective for patients medically deemed to contract any type of immunodeficiency or immunosuppression.

The desired lymphocytes of the patient can be gathered from peripheral blood, lymph nodes, or the thymus of the patient. Any autologous lymphocytes gathered from any tissues or organs of the patient may be efficiently used. Particularly, the lymphocytes of the peripheral blood from the veins of the patient are serviceable, because the blood can easily be drawn therefrom without imposing a burden on the patient. For the purpose of preventing the peripheral blood from coagulating, there may be added heparin or citric acid thereto, but this measure is not absolutely necessary to this invention. In this embodiment, separation of the desired lymphocytes from the peripheral blood is carried out by a density centrifugation method. The density centrifugation is fulfilled by use of Ficoll-Hypaque, monopoly resolution or the like, but these separation methods should not be understood as being limitative.

The blood may be gathered at a time on the order of 0.1 to 500 ml. It is desirable to gather 1 to 100 ml of blood, and more preferably 10 to 50 ml. The method according to this invention can be performed by use of a large amount of blood or even a small amount of blood. In the case of drawing a little bit of blood, a burden on the patient can be reduced to a minimum. Thus, the blood of the order of 50 ml may preferably be gathered at a time. From blood gathered at a time, desired cells available for several times of administering can be prepared, but may preferably be prepared by gathering the blood little by little several times in accordance with the number or interval of administering.

Any antibody capable of recognizing CD3 may be used as the anti-CD3 antibody. As one example, OKT3 antibody (made by Ortho Pharmaceutical Corp.) can be used. As a supporter for making the anti-CD3 antibody into a solid phase, various types of flasks, culture dishes, plates and bags may be used. A solid-phase method for antibody can be performed by non-specific adsorption or chemical bonding. However, the solid-phase method in this invention should not be limited thereto. Any solid-phase method capable of stimulating lymphocytes with the anti-CD3 antibody may be adopted. Further, any type of interleukin-2 may be used insofar as it can activate lymphocytes, particularly, T cells. The cultivation of cells in in-vitro can be effected by use of RPMI 1640 culture medium, DMEM culture medium, or non-serum culture medium. To the culture medium, there may be added blood serum, protein, amino acid, saccharide and/or antibiotic to cultivate the lymphocytes.

The lymphocytes cultivated in the in-vitro may be suspended in a buffer solution such as physiological saline and phosphate buffer solution to be administered to the patient. The lymphocytes having the cell concentration in the range of 1 × 10⁴ parts/lit. to 1 × 10⁸ parts/lit. may be used. It is preferable to use the lymphocytes having the cell concentration in the range of 1 × 10⁶ parts/lit. to 1 × 10⁸ parts/lit. It is desirable to add protein and so on to the cell-suspended solution to prevent the cells from coagulating and adhering to a receptacle. As the protein applied thereto, human albumin may be used in concentrations of from 0.1 w/v% to 10 w/v%, for instance.

Although the remedies/preventives of the invention may be administered to the patient through any administering route such as the abdominal cavity, artery, muscle and subcutaneous tissue, it is recommended to administer the remedies/preventives through the vein for the convenience of surgery. The administration of the remedies/preventives may be carried out when or before showing the infections. In this embodiment, the administration of the remedies/preventives is effected when showing the infections, since the curative effect of the remedies of the present invention can clearly be confirmed at that time. It would be surmised by a person skilled in the field that the agents having the curative effect have the preventive effect on the infections as a matter of course. Thus, the remedies/preventives according to the present invention can be used for not only remedying the infections, but also preventing the infections.

For giving the remedies or preventives to the patient, the lymphocytes proliferated by being stimulated with the anti-CD3 antibody made into a solid phase and interleukin-2 or the lymphocytes expansively cultivated in a suitable culture medium after providing stimulation thereto may be used. To the culture medium for expansively cultivating the lymphocytes, the interleukin-2 may be added according to need. Although the lymphocytes gathered in this invention are cultivated in the culture medium containing the anti-CD3 antibody made into a solid phase and interleukin-2 so as to be proliferated, various cytokines or proliferating factors may be added thereto, or the lymphocytes may be stimulated with a specific antigen to be proliferated.

Some clinical embodiments to which this invention was applied will be described hereinafter, but this invention does not contemplate imposing any limitation on the conditions of these examples.

### [EMBODIMENT 1] PREPARATION OF A FLASK FOR MAKING OKT3 ANTIBODY INTO A SOLID PHASE

To a cultivating flask (made by Sumitomo Bakelite Co., Ltd.) having a bottom area of 225 cm², 30 ml of PBS(-) solution containing OKT3 antibody having a concentration of 5 µg/ml was added. The flask having its bottom soaked in the solution was left for 2 hours at room temperature. Thus, an OKT3-solidifying flask for making OKT3 antibody into a solid phase was prepared and retained for use at 4°C.

### [EMBODIMENT 2] PREPARATION OF LYMPHOCYTES

From the vein of a patient contracting the Wiskott-Aldrich syndrome being the herpes simplex infections and EB viral infections, 50 ml of blood was drawn as heparin-added peripheral blood. To the blood thus dranw, RPMI 1640 culture medium of the same quantity as the blood was added. The blood with the culture medium was superposed on Lymphosepar-I (made by Immuno Biological Laboratory) distributed into several centrifugal beakers each having a capacity of 15 ml, and then, exposed to centrifugal force at 1800rpm for 15 minutes. Thereafter, the lymphocytes in the layered state were gathered with a pipette and mingled with RPMI 1640 culture medium, and then, subjected to centrifugal separation (at 1800 rpm for 10 minutes). Upon removing the top layer of each beaker, cell pellets thus obtained were thoroughly loosened. And then, to the cell pellets, there was added 50 ml of culture solution (obtained by adding 10% of human serum and 700 U/ml of interleukin-2 to the RPMI 1640 culture medium containing 60 µg/ml of kanamycin, 20 µg/ml of streptomycin, 2 mM of glutamine, 1 mM of oxaloacetic acid, 1 mM of pyruvate acid sodium, 10 mM of HEPES, and 0.2 U/ml of insulin), thereby to prepare a cell-suspended solution.

The OKT3-solidifying flask prepared in Embodiment 1 was washed with PBS(-) two times. After washing, the aforementioned cell-suspended solution was inoculated into the OKT3-solidifying flask and cultivated in the atmosphere of 5% of carbonic acid gas in saturated humidity at 37 °C. After 3 days, 50 ml of culture solution was added thereto, and further, after 4 days, 150 ml of culture solution was added. Upon suspending the culture solution, 125 ml of culture solution in the flask was put into another OKT3-solidifying flask to be cultivated. After 6 days, 250 ml of culture solution in the flask was mixed with 750 ml of LL-7 culture solution (made by Nikken Bio Medical Laboratory), and then, cultivated with a gas-penetrative cultivating bag in the atmosphere of 5% of carbonic acid gas at 37 °C. Eight days after the commencement of the experiment, 1 liter of culture solution was added and cultivated with two gas-penetrative cultivating bags. In the same manner, ten days after the commencement, the culture solution was cultivated with four bags, and then, twelve days after the commencement, it was cultivated with six bags. Fourteen days after the commencement, cells were gathered by a centrifugal separation process and suspended in about 250 ml of physiological saline containing 2% of human albumin, consequently to obtain a lymphocyte-suspended solution.

### [EMBODIMENT 3] ADMINISTRATION OF LYMPHOCYTES

The lymphocyte-suspended solution obtained in Embodiment 2 was infused into the vein of the patient five times. For each infusion, about 1.2 × 10¹⁰ of lymphocytes were used. Consequently, the affected focus in the patient's eye due to the herpes simplex infections was cured. The result thereof revealed that the lymphocytes are efficacious against viral infections caused by EB viruses.

### [EMBODIMENT 4] MEASUREMENT OF EB VIRUSES IN BLOOD

From the result of measuring, by a PCR measuring method, EB viruses in the blood of the patient to which the lymphocytes used in Embodiment 3 were applied, it was ascertained that the EB viruses in the blood are decreased with increasing the number of administration of the lymphocytes. As a result, the effect of the lymphocytes against the viral infections caused by EB viruses became evident.

### [EMBODIMENT 5]

Different lymphocytes were prepared and cultivated in the same manner as that in Embodiment 2 noted above, except that the blood was drawn from the vein of an HIV infected patient instead of drawing the blood from the vein of the patient contracting the Wiskott-Aldrich syndrome. Similarly to the lymphocytes of the patient contracting the Wiskott-Aldrich syndrome, the lymphocytes of the HIV infected patient could be proliferated, and fourteen days after the commencement of this experiment, the number of cells thereof reached 9 × 10⁹.

### INDUSTRIAL APPLICABILITY

According to this invention, the lymphocytes gathered from a patient are proliferated by being cultivated in the culture medium containing the anti-CD3 antibody and interleukin-2 made into a solid phase. Thus, there can be provided remedies or preventives for viral infections, which contain as the main ingredient, the obtained autologous lymphocytes cultivated in a culture medium containing an anti-CD3 antibody and interleukin-2 made into a solid phase, and a method for preparing the remedies or preventives according to the invention. Furthermore, the therapeutic method by use of the remedies or preventives to be administered to a patient contracting the infections according to the invention can not only produce noticeable curative effects of treatment and prevention of viral infections, but also effectively prevent postoperative or post-transplantation viral infections.

Since the remedies and preventives according to this invention are effective agains the viral infections which are exceedingly difficult of treatment and prevention, they can also be used for treating or preventing bacilli, mycosis, protozoa and so on. Besides, the present invention makes it possible to sort out a specific type of lymphocytes from the remedies or preventives of the invention, add certain cells the remedies or preventives of the invention, and activate the remedies and preventives of the invention with cytokines such as interleukin-12 for use in the treatment or prevention of the viral infections. By giving the remedies or preventives of the invention to a patient, the viral infections can be effectively treated or prevented by using jointly cytokines such as the interleukin-12 and various antiviral agents.

## Claims

1. Use of autologous lymphocytes from a patient having a viral infection and which lymphocytes are proliferated by being cultivated in a culture medium containing a solid phase anti-CD3 antibody and soluble interleukin-2, in the manufacture of a medicament for treating or preventing viral infections.

2. Use according to Claim 1, wherein said autologous lymphocytes are derived from a patient with immunodeficiency or immunosuppression.

## Patentansprüche

1. Verwendung von autologen Lymphozyten aus einem Patienten mit einer Virusinfektion und wobei die Lymphozyten proliferiert werden, indem sie in einem Kulturmedium, das einen Festphasen-anti-CD3-Antikörper und lösliches Interleukin-2 enthält, kultiviert werden, bei der Herstellung eines Medikaments zur Behandlung oder Prävention von Virusinfektionen.

2. Verwendung nach Anspruch 1, wobei die autologen Lymphozyten von einem Patienten mit Immundefizienz oder Immunsuppresion stammen.

## Revendications

1. Utilisation de lymphocytes autologues provenant d'un patient ayant une infection virale, lymphocytes que l'on fait proliférer en les cultivant dans un milieu de culture contenant un anticorps anti-CD3 en phase solide et de l'interleukine-2 soluble, dans la fabrication d'un médicament destiné à traiter ou prévenir des infections virales.

2. Utilisation selon la revendication 1, dans laquelle les lymphocytes autologues sont dérivés d'un patient présentant une immunodéficience ou une immunosuppression.
